# FASCICULE DE BREVET EUROPEEN

(11) **EP 0 931 542 B1**
(45) Date de publication et mention de la délivrance du brevet: **14.01.2004**
(21) Numéro de dépôt: 98403230.0
(22) Date de dépôt: 18.12.1998
(51) Int. Cl.: A61K 7/48

(54) **Composition cosmétique contenant du phloroglucinol**
Kosmetische Zusammensetzung, die Phloroglucinol enthält
Cosmetic composition containing phloroglucinol

(30) Priorité: 19.12.1997 FR 9716181
(43) Date de publication de la demande: 28.07.1999
(73) Titulaire: L'OREAL, 75008 Paris (FR)
(72) Inventeur: Breton, Lionel, 78000 Versailles (FR); Girerd-Dugue, Florence, 34130 Maugio (FR); Renault, Béatrice, 94410 Saint Maurice (FR)
(74) Mandataire: Tezier Herman, Béatrice

(56) Documents cités:
- EP-A- 0 716 847
- FR-A- 1 472 078
- CHEMICAL ABSTRACTS, vol. 121, no. 12, 19 septembre 1994 Columbus, Ohio, US; abstract no. 141243, ONODERA, JUNICHI ET AL: "Cosmetics containing phloroglucinol derivatives" XP002082502 & JP 06 092835 A (DAIICHI SEIYAKU CO, JAPAN)
- CHEMICAL ABSTRACTS, vol. 124, no. 14, 1 avril 1996 Columbus, Ohio, US; abstract no. 185112, OKAMOTO, NOBUO ET AL: "Scavenging activities of polyhydroxybenzoic acids on hydroxyl radicals" XP002082503 & J. SCCJ (1995), 29(3), 270-3 CODEN: JOSCDQ;ISSN: 0387-5253,1995,
- B.J. KIM: "Biological screening of 100 plant extracts for cosmetic use: (II): anti-oxidative activity and free radical scavening activity" INTERNATIONAL JOURNAL OF COSMETIC SCIENCE, vol. 19, 1997, pages 299-307, XP002084217
- CHEMICAL ABSTRACTS, vol. 122, no. 12, 20 mars 1995 Columbus, Ohio, US; abstract no. 142511, TAGASHIRA, MOTOYUKI: "Pharmaceuticals, foods, and cosmetics containing antioxidative humulones" XP002082506 & JP 06 312924 A (ASAHI BREWERIES LTD, JAPAN)
- DATABASE WPI Week 9539 Derwent Publications Ltd., London, GB; AN 95-299528 XP002082507 "New alkyl derivs. of beta-acid(s) e.g. lupulone - are used as antioxidants e.g. for treating rheumatoid arthritis or skin disorders or as a food preservative" A & JP 07 196572 A (ASAHI BREWERIES) 1 août 1995
- CHEMICAL ABSTRACTS, vol. 118, no. 2, 11 janvier 1993 Columbus, Ohio, US; abstract no. 11521, MITANI, YASUMASA ET AL: "1,3,5-Trihydroxybenzenes as tyrosinase inhibitors" XP002082504 & JP 04 235112 A (TAC GIJUTSU KAGAKU KENKYUSHO K. K., JAPAN)
- CHEMICAL ABSTRACTS, vol. 111, no. 20, 13 novembre 1989 Columbus, Ohio, US; abstract no. 180506, HAYASHI, RIEKO ET AL: "Deodorant compositions containing phloroglucinol polymers" XP002082505 & JP 01 090118 A (LION CORP., JAPAN)
- P. ROVESTI: "Recherches sur l'action des auxines et des phytohormones en cosmétique" PARFUMERIE MOD., vol. 48, no. 54, 1956, pages 86-91, XP002082741

## Description

L'invention a pour objet une composition cosmétique comprenant dans un milieu cosmétiquement acceptable au moins du phloroglucinol ou l'un de ses dérivés. L'invention a également pour objet l'utilisation du phloroglucinol ou de ses dérivés dans une composition cosmétique destinée à favoriser le renouvellement épidermique.

La peau constitue une barrière physique entre l'organisme et son environnement. Elle est constituée de deux tissus : l'épiderme et le derme.

Le derme fournit à l'épiderme un support solide. C'est également son élément nourricier. Il est principalement constitué de fibroblastes et d'une matrice extracellulaire composée elle-même principalement de collagène, d'élastine et d'une substance dite substance fondamentale, ces composants étant synthétisés par le fibroblaste. On y trouve aussi des leucocytes, des mastocytes ou encore des macrophages tissulaires. Il contient également de vaisseaux sanguins et des fibres nerveuses.

L'épiderme est un épithélium pluristratifié desquamant, de 100 µm d'épaisseur en moyenne et est conventionnellement divisé en une couche basale de kératinocytes qui constitue la couche germinative de l'épiderme, une couche dite épineuse constituée de plusieurs couches de cellules polyédriques disposées sur les cellules germinatives, une couche dite granuleuse constituée de cellules aplaties contenant des inclusions cytoplasmiques distinctes, les grains de kératohyaline, et enfin une couche supérieure appelée couche cornée (ou stratum corneum), constituée de kératinocytes au stade terminal de leur différenciation appelés cornéocytes. Ceux-ci sont des cellules momifiées, anucléées qui dérivent des kératinocytes. L'empilement de ces cornéocytes constitue la couche cornée qui est responsable entre autres de la fonction de barrière de l'épiderme.

La différenciation épidermique suit un processus de maturation continu et orienté dans lequel les kératinocytes basaux se transforment en migrant pour aboutir à la formation de cornéocytes, cellules mortes totalement kératinisées. Cette différenciation est la résultante de phénomènes parfaitement coordonnés qui vont conduire au maintien d'une épaisseur constante et assurer ainsi l'homéostasie de l'épiderme. Celle-ci passe par une régulation du nombre de cellules qui entrent dans le processus de différenciation et du nombre de cellules qui desquament. Au cours du processus normal de desquamation, seuls les cornéocytes les plus superficiels se détachent de la surface de l'épiderme.

On sait qu'au cours du vieillissement chronobiologique, l'épaisseur de l'épiderme se réduit. Les divisions cellulaires de la couche basale diminuent en nombre. Le temps de renouvellement des cellules cornées se rallonge. La maturation de ces cellules est imparfaite et la kératinisation n'aboutit plus à créer une couche cornée régulière et homogène.
On sait aussi que des expositions prolongées et/ou répétées au soleil aboutissent à des résultats assez similaires sur l'épiderme. C'est le vieillissement photo-induit. On sait aussi que dans le cas de certaines maladies comme l'ichtyose la peau subit des dommages par défaut de prolifération cellulaire.

On sait aussi qu'à la ménopause le vieillissement cutané s'accélère, l'épaisseur de la peau diminue, les femmes se plaignent de ce que leur peau tire et qu'elle prend l'aspect d'une "peau sèche", voire de l'apparition d'une xérose. On sait que les déficits hormonaux associés à la ménopause s'accompagnent d'un ralentissement général du métabolisme cellulaire, d'où on peut supposer que les effets que ressent la femme sont liés notamment à une diminution de la prolifération des kératinocytes.

On comprend alors la nécessité de disposer de moyen facilitant la multiplication cellulaire, particulièrement la multiplication des cellules de l'épiderme. En effet de tels moyens permettent de faciliter la régénération de l'épiderme et de redonner à la peau un aspect jeune.

On mesure également combien de tels produits pourraient être utiles dans le cas de la cicatrisation.

De manière surprenante et inattendue, la demanderesse à maintenant découvert que le phloroglucinol ou ses dérivés ont la propriété d'induire la prolifération des kératinocytes humains normaux.

Le phloroglucinol ou 1,3,5-trihydroxybenzène répond à la formule :

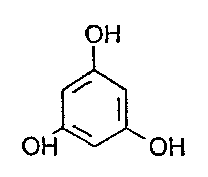

Il est présent par exemple dans des extraits végétaux comme par exemple ceux d'*Eucalyptus kino* ou d'*Acacia arabica.*

Par dérivés du phloroglucinol on entend ceux citer dans " Dictionary of Natural Products on CD-ROM" (Chapman et Hall (1997), London) comme le 1,3,5-triméthoxybenzène, le 1,3,5-triéthoxybenzène, le 3,5-diméthoxyphénol ou taxicatigénine , le 5-méthoxy-1,3-benzènediol ou flaménol, le 3-hydroxy-5-méthoxyphényl, le 1,3,5-triphenoxybenzène, le tribenzyléther, le O-β-glucopyranoside ou Phlorine, la taxicatine ou diméthoxyphényléther O-β-glucopyranoside, ou encore ceux cités dans "The organic constituents of higher plants (T. Robinson, 1983, Corduss press, North Amerst) comme les tautomères du phloroglucinol (tricétones cycliques), la désaspidine, la lupulone, l'humulone, le céropténe, la félicitine, la leptospermone, l'eugénone, l'hulupone, l'aspidinol, l'α-kosine, tasmanone ou encore l'angustione.

Ces dérivés sont présents dans une grande variété de végétaux et peuvent en être extraits par des méthodes classiques. On citera par exemple les végétaux de la famille des Pteridacées *(Asnidium),* des Cannabinacées *(Humulus lupulus),* des Myrtacées *(Eugenia caryophyllata, Eucalyptus risdoni).*

Dans l'art antérieur le phloroglucinol ou ses dérivés sont connus comme inhibiteurs de transport photosynthétique des électrons, comme inhibiteur de la promotion tumorale, comme inhibiteurs de la photophosphorylation ou comme inhibiteur du métabolisme de l'acide arachidonique. Ce sont également des antihelmintiques, des agents spasmolitiques, des insecticides, des bactéricides ou encore surtout des colorants, particulièrement utilisés pour la teinture des tissus.

A la connaissance de la demanderesse il n'a jamais été décrit dans l'état de la technique que le phloroglucinol ou ses dérivés stimule la prolifération des kératinocytes.

L'invention a donc pour objet une composition cosmétique comprenant dans un milieu cosmétiquement acceptable une quantité efficace d'au moins un dérivé de phloroglucinol.

Selon l'invention, le phloroglucinol ou ses dérivés peuvent être d'origine naturelle ou synthétique. Par origine naturelle, on entend le phloroglucinol, ou ses dérivés, préparés à partir de matériel végétal dans lequel ils se trouvent présents à l'état naturel. Par origine synthétique on entend le phloroglucinol, ou ses dérivés, préparés par synthèse chimique ou par biotechnologie.
Ainsi, par la suite dans le texte le terme phloroglucinol s'entend comme désignant du phloroglucinol ou ses dérivés, d'origine naturelle ou synthétique, purifiés ou toute préparation les contenant.

Préférentiellement, selon l'invention on utilise le phloroglucinol ou le 1,3,5-triméthoxybenzène.

Bien entendu, il est possible d'utiliser selon l'invention le phloroglucinol ou ses dérivés seuls ou en mélange.

On a vu précédemment qu'au cours du vieillissement chronobiologique et/ou photo-induit, la taille de l'épiderme diminuait principalement sous l'effet d'une réduction du nombre des divisions cellulaires de la couche basale et d'un allongement du temps de renouvellement des cellules cornées.

Ainsi, un des aspects de l'invention est donc de proposer une composition cosmétique destinée à stimuler la prolifération des kératinocytes de la peau comprenant dans un milieu cosmétiquement acceptable une quantité efficace de phloroglucinol ou de l'un de ses dérivés.

L'invention a également pour objet une composition cosmétique destinée à lutter contre le vieillissement chronobiologique et/ou photo-induit comprenant dans un milieu cosmétiquement acceptable une quantité efficace de phloroglucinol ou de l'un de ses dérivés.

L'invention a encore pour objet une composition cosmétique destinée à stimuler la cicatrisation comprenant dans un milieu cosmétiquement acceptable une quantité efficace de phloroglucinol ou de l'un de ses dérivés.

L'invention a enfin pour objet une composition cosmétique destinée à combattre les effets cutanés de la ménopause, comprenant dans un milieu cosmétiquement acceptable une quantité efficace de phloroglucinol ou de l'un de ses dérivés.

Par milieu cosmétiquement acceptable, on entend compatible avec la peau, le cuir chevelu, les muqueuses, les ongles et les cheveux.

La quantité de phloroglucinol utilisable selon l'invention dépend bien évidemment de l'effet recherché et doit être en une quantité efficace pour stimuler la prolifération des kératinocytes.

A titre d'exemple la quantité de phloroglucinol ou de ses dérivés utilisable selon l'invention peut aller par exemple de 0,0001% à 5% et de préférence de 0,001 % à 2% du poids total de la composition.

La peau étant constituée de bien d'autres composants que les kératinocytes, il s'avère intéressant, lorsque l'on utilise du phloroglucinol ou l'un de ses dérivés selon l'invention, de favoriser en même temps la synthèse de ces autres composants comme par exemple le collagène et/ou les lipides.

Ainsi, l'invention a pour objet une composition cosmétique comprenant dans un milieu cosmétiquement acceptable du phloroglucinol ou l'un de ses dérivés et au moins un autre produit stimulant la synthèse du collagène et/ou la synthèse des lipides.

A cet égard on peut citer comme produit stimulant la synthèse du collagène et/ou la synthèse des lipides les hormones végétales, comme les auxines, ou des composés d'origine végétale, comme l'acide cinnamique.

Ainsi, les compositions selon l'invention peuvent comprendre en plus du phloroglucinol ou de l'un de ses dérivés, de l'acide cinnamique ou ses dérivés et/ou une hormone végétale, particulièrement une auxine choisie parmi l'acide indolacétique (IAA), l'acide 4-chloroindole-3-acétique (4-Cl-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'indole acétaldéhyde et l'indole acétonitrile.

Préférentiellement, selon l'invention la composition comprend du phloroglucinol ou l'un de ses dérivés et de l'acide cinnamique et/ou de l'acide β-naphtoxyacétique. Par exemple, la composition comprend du 1,3,5-triméthoxybenzène, de l'acide β-naphtoacétique et de l'acide cinnamique.

Comme produit stimulant la synthèse du collagène, on peut aussi citer la vitamine C et ses dérivés.

Dans les compositions selon l'invention, le produit stimulant la synthèse des lipides et/ou du collagène peut être en une quantité comprise entre 10⁻⁶% et 10%, et de préférence entre 10⁻³% et 5% du poids total de la composition.

En outre, l'invention a pour objet l'utilisation dans une composition cosmétique dans un milieu cosmétiquement acceptable d'une quantité efficace de phloroglucinol ou de ses dérivés, le phloroglucinol ou la composition étant destiné à stimuler la prolifération des kératinocytes et ainsi à favoriser la régénération de la peau.

De même, un des aspects de l'invention est de proposer l'utilisation dans une composition cosmétique dans un milieu cosmétiquement acceptable d'une quantité efficace de phloroglucinol ou de ses dérivés, le phloroglucinol ou la composition étant destiné à lutter contre le vieillissement chronobiologique et/ou photo-induit.

Sous un autre aspect encore, l'invention a pour objet l'utilisation dans une composition cosmétique dans un milieu cosmétiquement acceptable d'une quantité efficace de phloroglucinol ou de ses dérivés, le phloroglucinol ou la composition étant destiné à favoriser la cicatrisation.

Sous un autre aspect encore, l'invention a pour objet l'utilisation dans une composition cosmétique dans un milieu cosmétiquement acceptable d'une quantité efficace de phloroglucinol ou de ses dérivés, le phloroglucinol ou la composition étant destiné à lutter contre les effets cutanés de la ménopause, particulièrement les effets de la ménopause sur la prolifération des kératinocytes.

La composition selon l'invention comprend bien évidemment un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologiques, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser selon l'invention en association avec du phloroglucinol ou de l'un de ses dérivés, des composés choisis parmi:
- les hormones végétales;
- les agents antibactériens tels que les macrolides, les pyranosides et les tétracyclines, et notamment l'érythromycine ;
- les agents antagonistes de calcium, comme le vérapamil et le diltiazem ;
- des capteurs de radicaux OH, tels que le diméthylsulfoxyde ;
- des extraits de végétaux tels que ceux d'Iridacés ou de soja, extraits pouvant alors contenir ou non des isoflavones ;
- des extraits de micro-organismes dont en particulier des extraits bactériens comme ceux de bactéries filamenteuses non photosynthétiques.

A la liste ci-dessus, d'autres composés peuvent également être ajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, les lactones et leurs sels correspondants, l'anthraline, des caroténoides, les acides eicosatétraénoïque et eicosatriénoïque ou leurs esters et amides, la vitamine D et ses dérivés.

Selon l'invention, on peut, entre autres, associer le phloroglucinol ou l'un de ses dérivés à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les agents modulant l'adhésion bactérienne sur la peau et /ou les muqueuses tels que le miel, notamment le miel d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Ainsi, un autre objet de l'invention concerne une composition comprenant une quantité efficace de phloroglucinol ou de l'un de ses dérivés et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

Bien entendu le phloroglucinol, ou l'un de ses dérivés, peut être utilisé dans la préparation de compositions cosmétiques et/ou pharmaceutiques, particulièrement dermatologiques, destinées à stimuler la prolifération des kératinocytes.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemple 1: Etude de l'effet du 1,3,5-triméthoxybenzène sur la prolifération des kératinocytes humains normaux en culture en monocouche :

### Matériel et méthodes:

### Cellules

Les produits sont testés sur des kératinocytes humains normaux de 2^{ième} passage cultivés en milieu KGM (milieu défini sans sérum vendu par la société Clonetics).
La gamme de concentrations est comprise entre 10⁻⁴M et 10⁻⁷M.
Les produits sont testés 2 fois sur 2 plaques différentes.

### Témoin

L'épinéphrine à 250ng/ml est employée comme témoin positif de la prolifération.

### Mise en solution du produit:

Les produits sont solubilisés dans le diméthylsulfoxyde (DMSO). Une solution mère à 1x10⁻²M est faite en DMSO. Les dilutions suivantes sont réalisées en KGM.
Les kératinocytes sont ensemencés à 6000 cellules/cm². Après 24h de culture le produit est appliqué sur les cellules pendant 72h. Le milieu est renouvelé après 48h de traitement.

### Test BrdU:

Kit BrdU (Boehringer Mannheim).
Le deuxième jour de traitement un analogue de la thymidine (BrdU) est ajouté dans le milieu de culture sur la nuit. Cet analogue permet de marquer les cellules en cours de synthèse d'ADN. Le troisième jour de traitement les cellules ayant incorporé du BrdU sont identifiées par un anticorps anti BrdU.

### Test au Bleu Alamar:

Le Bleu Alamar mesure l'activité métabolique des cellules. L'activité métabolique des cellules entraîne la réduction du milieu de culture qui se traduit par le virage de ce dernier du bleu au rose.
Le troisième jour du traitement les cellules sont mises en contact avec le Bleu Alamar dilué au 1/10 dans du KGM. Les cellules sont incubées durant 6h puis la densité optique (D.O.) est faite directement sur le surnageant de culture.

### Résultats:

Pour chaque produit un score (voir tableau) est attribué en fonction du résultat obtenu par rapport à l'épinéphrine (témoin positif pour la prolifération).

| **Valeur de DO** | **Score attribué** |
|---|---|
| Produit > Epinéphrine | 3 |
| Produit = Epinéphrine | 2 |
| Produit < Epinéphrine mais > Témoin culture | 1 |
| Produit = Témoin culture | 0 |
| Produit < Témoin culture | -1 |

1) Cytotoxicité des produits (Test au Bleu Alamar)
   De 10⁻⁵M à 10⁻⁷M , aucun effet cytotoxique marqué n'est observé.
2) Effet prolifératif du 1, 3, 5 triméthoxybenzéne :
   Le 1, 3, 5 triméthoxybenzène induit une prolifération des kératinocytes à 10⁻⁵M.

Conclusion : Le phloroglucinol induit la prolifération des kératinocytes humains normaux en monocouche avec un score de 1.

### Exemple 2 : Exemples de compositions selon l'invention. Ces compositions sont obtenues par les techniques habituelles couramment utilisées en cosmétique ou en pharmacie.

| Composition n° 1 | |
|---|---|
| Cire d'abeille | 1,5 % |
| Huile d'amandes d'abricot | 13,0 % |
| Conservateurs | 0,3 % |
| Parfum | 0,4 % |
| Triméthoxybenzène | 0,01 % |
| Xanthane | 0,5 % |
| Cyclopenta diméthylsiloxane | 5,0 % |
| Eau déminéralisée stérilisée | 69,29 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n° 2 | |
|---|---|
| Cire d'abeille | 1,5 % |
| Huile d'amandes d'abricot | 13,0 % |
| Conservateurs | 0,3 % |
| Parfum | 0,4 % |
| Triméthoxybenzène | 0,01 % |
| Acide cinnamique | 0,01 % |
| Xanthane | 0,5 % |
| Cyclopenta diméthylsiloxane | 5,0 % |
| Eau déminéralisée stérilisée | 69,28 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n° 3 | |
|---|---|
| Cire d'abeille | 1,5 % |
| Huile d'amandes d'abricot | 13,0 % |
| Conservateurs | 0,3 % |
| Parfum | 0,4 % |
| Triméthoxybenzène | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrase | 3,0 % |
| Sesquistéarate de méthylglucose | 3,0 % |
| Acide stéarique | 1,0 % |
| Alcool cétylique | 3,0 % |

| Composition n°4 | |
|---|---|
| Cire d'abeille | 1,5 % |
| Huile d'amandes d'abricot | 13,0 % |
| Conservateurs | 0,3 % |
| Parfum | 0,4 % |
| Leptospermone | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n°5 | |
|---|---|
| Cire d'abeille | 1,5 % |
| Huile d'amandes d'abricot | 13,0 % |
| Conservateurs | 0,3 % |
| Parfum | 0,4 % |
| Eugénone | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n° 6 | |
|---|---|
| Cire d'abeille | 1,5 % |
| Huile d'amandes d'abricot | 13,0 % |
| Conservateurs | 0,3 % |
| Parfum | 0,4 % |
| Hulupone | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n° 7 | |
|---|---|
| Cire d'abeille | 1,5 % |
| Huile d'amandes d'abricot | 13,0 % |
| Conservateurs | 0,3 % |
| Parfum | 0,4 % |
| Céroptène | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 68,77 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |
| Extrait de soja | 0,5 % |

| Composition n°8 | |
|---|---|
| Cire d'abeille | 1,5 % |
| Huile d'amandes d'abricot | 13,0 % |
| Conservateurs | 0,3 % |
| Parfum | 0,4 % |
| Aspidinol | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n°9 | |
|---|---|
| Cire d'abeille | 1,5 % |
| Huile d'amandes d'abricot | 13,0 % |
| Conservateurs | 0,3 % |
| Parfum | 0,4 % |
| Tasmanone | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27 % |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

| Composition n° 10 | |
|---|---|
| Cire d'abeille | 1,5 % |
| Huile d'amandes d'abricot | 13,0 % |
| Conservateurs | 0,3 % |
| Parfum | 0,4 % |
| Angustione | 0,01 % |
| Acide cinnamique | 0,01 % |
| Acide β-naphtoxyacétique | 0,01 % |
| Xanthane | 0,5 % |
| Cyclopenta diméthylsiloxane | 5,0 % |
| Eau déminéralisée stérilisée | 69,27% |
| Mono-di-palmitostéarate de sucrose | 3,0 % |
| Sesquistéarate de méthylglucose | 3,0 % |
| Acide stéarique | 1,0 % |
| Alcool cétylique | 3,0 % |

## Revendications

1. Utilisation non thérapeutique dans une composition cosmétique d'une quantité efficace de phloroglucinol ou d'au moins l'un de ses dérivés, le phloroglucinol ou la composition étant destiné à stimuler la prolifération des kératinocytes.

2. Utilisation selon la revendication 1 pour favoriser la régénération de la peau.

3. Utilisation selon la revendication 1 pour lutter contre le vieillissement chronobiologique et/ou photo-induit de la peau.

4. Utilisation selon la revendication 1 pour lutter contre les effets cutanés de la ménopause.

5. Utilisation selon l'une quelconque des revendications 1 à 4, **caractérisée par le fait que** les dérivés de phloroglucinol sont choisis parmi le 1,3,5-triméthoxybenzène, le 1,3,5-triéthoxybenzène, le 3,5-diméthoxyphénol ou taxicatigénine, le 5-méthoxy-1,3-benzenediol ou flaménol, le 3-hydroxy-5-méthoxyphényl, le 1,3,5-triphénoxybenzène, le tribenzyléther, le O-β-glucopyranoside ou phlorine, la taxicatine ou diméthoxyphényléther O-β-glucopyranoside, les tautomères du phloroglucinol (tricétones cycliques), la désaspidine, la lupulone, l'humutone, le céropténe, la félicitine, la leptospermone, l'eugénone, l'hulupone, l'aspidinol, l'α-kosine, la tasmanone ou encore l'angustione.

6. Utilisation selon l'une quelconque des revendications 1 à 5, **caractérisée par le fait que** l'on utilise le phloroglucinol ou le 1,3,5-triméthoxybenzène.

7. Utilisation selon l'une quelconque des revendications 1 à 6, **caractérisée par le fait que** le phloroglucinol ou son dérivé est présent en une quantité allant de 0,0001% à 5% du poids total de la composition.

8. Utilisation selon la revendication précédente, **caractérisée par le fait que** le phloroglucinol est présent en une quantité allant de 0,001 % à 2% du poids total de la composition.

9. Composition cosmétique susceptible d'être utilisée selon l'une des revendications 1 à 8 comprenant, dans un milieu cosmétiquement acceptable, une quantité efficace d'au moins un dérivé de phloroglucinol choisi parmi le 1,3,5-triméthoxybenzène, le 1,3,5-triéthoxybenzène, le 3,5-diméthoxyphénol ou taxicatigénine, le 5-méthoxy-1,3-benzenediol ou flaménol, le 3-hydroxy-5-méthoxyphényl, le 1,3,5-triphénoxybenzène, le tribenzyléther, la taxicatine ou diméthoxyphényléther O-β-glucopyranoside, les tautomères du phloroglucinol (tricétones cycliques), la désaspidine, le céropténe, la félicitine, la leptospermone, l'eugénone, l'hulupone, l'aspidinol, l'α-kosine, la tasmanone ou encore l'angustione.

10. Composition cosmétique susceptible d'être utilisée selon l'une des revendications 1 à 8 comprenant, dans un milieu cosmétiquement acceptable, une quantité efficace d'au moins du 1,3,5-triméthoxybenzène.

11. Composition selon l'une des revendications 9 ou 10, **caractérisée par le fait que** le dérivé du phloroglucinol est présent en une quantité allant de 0,0001% à 5% du poids total de la composition

12. Composition selon l'une des revendications 9 à 11, **caractérisée par le fait que** le dérivé du phloroglucinol est présent en une quantité allant de 0,001% à 2% du poids total de la composition.

13. Composition cosmétique susceptible d'être utilisée selon l'une des revendications 1 à 8 comprenant dans un milieu cosmétiquement acceptable une quantité efficace de phloroglucinol ou d'au moins un de ses dérivés et au moins un composé stimulant la synthèse des lipides et/ou du collagène.

14. Composition selon la revendication 13, **caractérisée par le fait que** l'autre produit stimulant la synthèse des lipides et/ou du collagène est choisi parmi les hormones végétales ou l'acide cinnamique et ses dérivés.

15. Composition selon la revendication précédente, **caractérisée par le fait que** l'hormone végétale est une auxine.

16. Composition selon la revendication précédente, **caractérisée par le fait que** l'auxine est choisie parmi l'acide indole-acétique (IAA), l'acide 4-chloroindole-3-acétique (4-Cl-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'indole acétaldéhyde et l'indole acétonitrile.

17. Composition selon la revendication précédente, **caractérisée par le fait que** l'auxine est de l'acide β-naphtoxyacétique

18. Composition selon la revendication 13, **caractérisée en ce que** l'autre produit stimulant la synthèse du collagène est choisi parmi la vitamine C ou ses dérivés.

19. Composition cosmétique selon l'une des revendications 9 à 18, **caractérisée par le fait qu'**elle comprend dans un milieu cosmétiquement acceptable du 1,3,5-triméthoxybenzène, de l'acide β-naphtoxyacétique et de l'acide cinnamique.

20. Composition selon l'une quelconque des revendications 13 à 19, **caractérisée par le fait que** l'autre produit stimulant la synthèse des lipides et/ou du collagène est en une quantité comprise entre 10⁻⁶% et 10%.

21. Composition selon la revendication précédente, **caractérisée par le fait que** l'autre produit stimulant la synthèse des lipides et/ou du collagène est en une quantité comprise entre 10⁻³% et 5%.

## Patentansprüche

1. Nicht therapeutische Verwendung von Phloroglucin oder mindestens einem seiner Derivate in einer wirksamen Menge in einer kosmetischen Zusammensetzung, wobei das Phloroglucin oder die Zusammensetzung dazu vorgesehen sind, die Proliferation der Keratinocyten zu stimulieren.

2. Verwendung nach Anspruch 1, um die Regenerierung der Haut zu fördern.

3. Verwendung nach Anspruch 1, um die altersbedingte und/oder photoinduzierte Hautalterung zu bekämpfen.

4. Verwendung nach Anspruch 1, um die Wirkungen der Menopause auf die Haut zu bekämpfen.

5. Verwendung nach einem der Ansprüche 1 bis 4, **dadurch gekennzeichnet, dass** die Derivate des Phloroglucin unter 1,3,5-Trimethoxybenzol, 1,3,5-Triethoxybenzol, 3,5-Dimethoxyphenol oder Taxicatigenin, 5-Methoxy-1,3-benzoldiol oder Flamenol, 3-Hydroxy-5-methoxyphenyl, 1,3,5-Triphenoxybenzol, Tribenzylether, O-β-Glucopyranosid oder Phlorin, Taxicatin oder Dimethoxyphenylether-O-β-glucopyranosid, Tautomeren von Phloroglucin (cyclischen Triketonen), Desaspidin, Lupulon, Humulon, Ceropten, Felicitin, Leptospermon, Eugenon, Hulupon, Aspidinol, α-Kosin, Tasmanon oder Angustion ausgewählt sind.

6. Verwendung nach einem der Ansprüche 1 bis 5, **dadurch gekennzeichnet, dass** das Phloroglucin oder das 1,3,5-Trimethoxybenzol verwendet wird.

7. Verwendung nach einem der Ansprüche 1 bis 6, **dadurch gekennzeichnet, dass** das Phloroglucin oder sein Derivat in einer Menge von 0,0001 bis 5 % des Gesamtgewichts der Zusammensetzung vorliegt.

8. Verwendung nach dem vorhergehenden Anspruch, **dadurch gekennzeichnet, dass** das Phloroglucin in einer Menge von 0,001 bis 2 % des Gesamtgewichts der Zusammensetzung vorliegt.

9. Kosmetische Zusammensetzung, die nach einem der Ansprüche 1 bis 8 verwendet werden kann und die in einem kosmetisch akzeptablen Medium eine wirksame Menge mindestens eines Phloroglucinderivats enthält, das unter 1,3,5-Trimethoxybenzol, 1,3,5-Triethoxybenzol, 3,5-Dimethoxyphenol oder Taxicatigenin, 5-Methoxy-1,3-benzoldiol oder Flamenol, 3-Hydroxy-5-methoxyphenyl, 1,3,5-Triphenoxybenzol, Tribenzylether, O-β-Glucopyranosid oder Phlorin, Taxicatin oder Dimethoxyphenylether-O-β-glucopyranosid, Tautomeren von Phloroglucin (cyclischen Triketonen), Desaspidin, Ceropten, Felicitin, Leptospermon, Eugenon, Hulupon, Aspidinol, α-Kosin, Tasmanon oder Angustion ausgewählt ist.

10. Kosmetische Zusammensetzung, die nach einem der Ansprüche 1 bis 8 verwendet werden kann und die in einem kosmetisch akzeptablen Medium zumindest das 1,3,5-Trimethoxybenzol in einer wirksamen Menge enthält.

11. Zusammensetzung nach einem der Ansprüche 9 oder 10, **dadurch gekennzeichnet, dass** das Phloroglucinderivat in einer Menge von 0,0001 bis 5 % des Gesamtgewichts der Zusammensetzung enthalten ist.

12. Zusammensetzung nach einem der Ansprüche 9 bis 11, **dadurch gekennzeichnet, dass** das Phloroglucinderivat in einer Menge von 0,001 bis 2 % des Gesamtgewichts der Zusammensetzung enthalten ist.

13. Kosmetische Zusammensetzung, die nach einem der Ansprüche 1 bis 8 verwendet werden kann und die in einem kosmetisch akzeptablen Medium eine wirksame Menge von Phloroglucin oder mindestens eines Phloroglucinderivats und mindestens eine Verbindung enthält, die die Synthese von Lipiden und/oder Kollagen stimuliert.

14. Zusammensetzung nach Anspruch 13, **dadurch gekennzeichnet, dass** das Produkt, das die Synthese von Lipiden und/ oder Kollagen stimuliert, unter den pflanzlichen Hormonen und Zimtsäure und ihren Derivaten ausgewählt ist.

15. Zusammensetzung nach dem vorhergehenden Anspruch, **dadurch gekennzeichnet, dass** es sich bei dem pflanzlichen Hormon um ein Auxin handelt.

16. Zusammensetzung nach dem vorhergehenden Anspruch, **dadurch gekennzeichnet, dass** das Auxin unter Indolessigsäure (IAA), 4-Chlor-3-indolessigsäure (4-Cl-IAA), Phenylessigsäure (PAA), 3-Indolbuttersäure (IBA), 2,4-Dichlorphenoxyessigsäure (2,4-D), α-Naphthalinessigsäure (α-NAA), β-Naphthoxyessigsäure, Indolethanol, Indolacetaldehyd und Indolacetonitril ausgewählt ist.

17. Zusammensetzung nach dem vorhergehenden Anspruch, **dadurch gekennzeichnet, dass** das Auxin die β-Naphthoxyessigsäure ist.

18. Zusammensetzung nach Anspruch 13, **dadurch gekennzeichnet, dass** das andere Produkt, das die Synthese von Kollagen stimuliert, unter Vitamin C und seinen Derivaten ausgewählt ist.

19. Kosmetische Zusammensetzung nach einem der Ansprüche 9 bis 18, **dadurch gekennzeichnet, dass** sie in einem kosmetisch akzeptablen Medium 1,3,5-Trimethoxybenzol, β-Naphthoxyessigsäure und Zimtsäure enthält.

20. Zusammensetzung nach einem der Ansprüche 10 bis 19, **dadurch gekennzeichnet, dass** das andere Produkt, das die Synthese von Lipiden und/oder Kollagen stimuliert, in einer Menge von 10⁻⁶ bis 10 % vorliegt.

21. Zusammensetzung nach dem vorhergehenden Anspruch, **dadurch gekennzeichnet, dass** das andere Produkt, das die Synthese von Lipiden und/oder Kollagen stimuliert, in einer Menge von 10⁻³ bis 5 % vorliegt.

## Claims

1. Non-therapeutic use in a cosmetic composition of an effective amount of phloroglucinol or of at least one of its derivatives, the phloroglucinol or the composition being intended to stimulate the proliferation of keratinocytes.

2. Use according to Claim 1 to promote the regeneration of the skin.

3. Use according to Claim 1 to combat chronobiological and/or photoinduced ageing of the skin.

4. Use according to Claim 1 to combat the cutaneous effects of the menopause.

5. Use according to any one of Claims 1 to 4, **characterized in that** the phloroglucinol derivatives are chosen from 1,3,5-trimethoxybenzene, 1,3,5-triethoxybenzene, 3,5-dimethoxyphenol or taxicatigenin, 5-methoxy-1,3-benzenediol or flamenol, 3-hydroxy-5-methoxyphenol, 1,3,5-triphenoxybenzene, the tribenzyl ether, the O-β-glucopyranoside or phlorin, taxicatin or dimethoxyphenyl O-β-glucopyranoside, the tautomers of phloroglucinol (cyclic triketones), desaspidin, lupulon, humulon, ceroptene, felicitin, leptospermone, eugenone, hulupon, aspidinol, α-kosin, tasmanone or angustione.

6. Use according to any one of Claims 1 to 5, **characterized in that** phloroglucinol or 1,3,5-trimethoxybenzene is used.

7. Use according to any one of Claims 1 to 6, **characterized in that** the phloroglucinol or its derivative is present in an amount ranging from 0.0001% to 5% of the total weight of the composition.

8. Use according to the preceding claim, **characterized in that** the phloroglucinol is present in an amount ranging from 0.001% to 2% of the total weight of the composition.

9. Cosmetic composition capable of being used according to one of claims 1 to 8, comprising, in a cosmetically acceptable medium, an effective amount of at least one phloroglucinol derivative chosen from 1,3,5-trimethoxybenzene, 1,3,5-triethoxybenzene, 3,5-dimethoxyphenol or taxicatigenin, 5-methoxy-1,3-benzenediol or flamenol, 3-hydroxy-5-methoxyphenol, 1,3,5-triphenoxybenzene, the tribenzyl ether, taxicatin or dimethoxyphenyl O-β-glucopyranoside, the tautomers of phloroglucinol (cyclic triketones), desaspidin, ceroptene, felicitin, leptospermone, eugenone; hulupon, aspidinol, α-kosin, tasmanone or angustione.

10. Cosmetic composition capable of being used according to one of claims 1 to 8, comprising, in a cosmetically acceptable medium, an effective amount of at least 1,3,5-trimethoxybenzene.

11. Composition according to either of claims 9 and 10, **characterized in that** the phloroglucinol derivative is present in an amount ranging from 0.0001% to 5% of the total weight of the composition.

12. Composition according to one of claims 9 to 11, **characterized in that** the phloroglucinol derivative is present in an amount ranging from 0.001% to 2% of the total weight of the composition.

13. Cosmetic composition capable of being used according to one of claims 1 to 8, comprising, in a cosmetically acceptable medium, an effective amount of phloroglucinol or of at least one of its derivatives and at least one compound which stimulates the synthesis of lipids and/or of collagen.

14. Composition according to Claim 13, **characterized in that** the other product which stimulates the synthesis of lipids and/or of collagen is chosen from plant hormones or cinnamic acid and its derivatives.

15. Composition according to the preceding claim, **characterized in that** the plant hormone is an auxin.

16. Composition according to the preceding claim, **characterized in that** the auxin is chosen from indoleacetic acid (IAA), 4-chloroindole-3-acetic acid (4-Cl-IAA), phenylacetic acid (PAA), indole-3-butyric acid (IBA), 2,4-dichlorophenoxyacetic acid (2,4-D), α-naphthaleneacetic acid (α-NAA), β-naphthoxyacetic acid, indoleethanol, indoleacetaldehyde and indoleacetonitrile.

17. Composition according to the preceding claim, **characterized in that** the auxin is β-naphthoxyacetic acid.

18. Composition according to Claim 13, **characterized in that** the other product which stimulates the synthesis of collagen is chosen from vitamin C or its derivatives.

19. Cosmetic composition according to one of Claims 9 to 18, **characterized in that** it comprises, in a cosmetically acceptable medium, 1,3,5-trimethoxybenzene, β-naphthoxyacetic acid and cinnamic acid.

20. Composition according to any one of Claims 10 to 19, **characterized in that** the other product which stimulates the synthesis of lipids and/or of collagen is in an amount of between 10⁻⁶% and 10%.

21. Composition according to the preceding claim, **characterized in that** the other product which stimulates the synthesis of lipids and/or of collagen is in an amount of between 10⁻³ % and 5%.
